# EUROPEAN PATENT APPLICATION

(11) **EP 4 134 977 A1**
(43) Date of publication of application: **15.02.2023**
(21) Application number: 21191275.3
(22) Date of filing: 13.08.2021
(51) Int. Cl.: G16H 50/20, G16H 50/70

(54) **PREDICTING RADIOLOGIC FINDINGS WITH MACHINE-LEARNING**

(71) Applicant: Siemens Healthcare GmbH, 91052 Erlangen (DE)
(72) Inventor: Comaniciu, Dorin, Princeton 08540 (US); Farri, Oladimeji, Upper Saddle River 07458 (US); Georgescu, Bogdan, Princeton 08540 (US); Ghesu, Florin-Cristian, Skillman 08558 (US); Mansoor, Awais, Potomac 20854 (US); Vunikili, Ramya, Secaucus 07094 (US)

(57) **Abstract**

The present invention relates to a method for predicting radiologic findings. In one embodiment, the method comprises obtaining (5), by a machine-learning model, text data representing at least one radiologic finding relating to a patient and, optionally, text data from an electronic medical record of the patient, and generating (6), by the machine-learning model, at least one predicted additional radiologic finding relating to the patient. The machine-learning model has been trained (3) using training data, the training data comprising text data from radiology reports and text data from electronic medical records of a plurality of patients.

## Description

The present invention relates generally to the field of computer-aided diagnosis (CAD), i.e., using computer-generated output as an assisting tool for a clinician to make a diagnosis, and more specifically to a method for predicting radiologic findings to enhance clinical decision making.

Medical imaging provides techniques for imaging the interior of a body, organ or tissue for clinical analysis and medical intervention, and is used e.g., in radiology to diagnose and treat diseases.

However, analyzing and interpreting medical images by a human radiologist can have its limits. For example, it is said that interpretation of radiology images that contain abnormalities by a radiologist can in some cases be associated with an error rate of about 30%. Furthermore, delayed diagnoses in radiology resulting from errors related to not recognizing abnormalities persist in 30% of cases despite subsequent examinations and review of images.

The types of errors that typically lead to delayed diagnoses in radiology can be grouped into three broad categories, namely underreading, faulty reasoning and satisfaction of search.

Satisfaction of search generally refers to the tendency of radiologists to discontinue searching for additional abnormalities after a first, often major, abnormality has been discovered. According to some research, satisfaction of search errors can be especially severe, accounting for 22% of errors in some scenarios (see Kim, Young W. et al. "Fool me twice: delayed diagnoses in radiology with emphasis on perpetuated errors", American journal of roentgenology 202.3 (2014): 465-470).

To address satisfaction of search errors in the prior art, typically a second reading is suggested, i.e., providing a more experienced radiologist access to the medical images after they have been read by a less experienced radiologist. In addition, relevant information from electronic medical records (EMR) of the patient who was scanned may be displayed simultaneously on the radiologist's computer for the radiologist to consider while interpreting the medical images. However, these approaches still rely on the radiologist's capabilities and are therefore prone to error and do not reliably decrease the rate of satisfaction of search errors.

Some prior art approaches, such as the one disclosed in US 9,934,567 B2, set out to improve this situation by training a model on images and biopsy data to guide the radiologist's interpretation using correlations with biopsy findings.

Other approaches, such as US 10,629,305 B2, retrain a computer-aided diagnosis (CAD) application on prior imaging findings or regions of interest (ROI), annotated by radiologists or otherwise, to improve the accuracy of detecting abnormalities in subsequent images.

Against this background, it is the technical problem underlying the present invention to provide methods and systems that enable a more precise medical diagnosis, in particular by decreasing the likelihood of satisfaction of search errors by the clinician, and therefore ultimately help to enhance clinical decision making.

This problem is in one embodiment solved by a computer-implemented method for predicting radiologic findings. The method may comprise obtaining, by a machine-learning model, text data representing at least one radiologic finding relating to a patient and, optionally, text data from an electronic medical record of the patient. Moreover, the method may comprise generating, by the machine-learning model, at least one predicted additional radiologic finding relating to the patient. The machine-learning model may be based on training data, the training data comprising text data from radiology reports and text data from electronic medical records of a plurality of patients. In particular, the machine-learning model may be based on training date by the machine-learning model having been trained using the training data using a training algorithm (e.g., the backpropagation algorithm).

Accordingly, one embodiment of the present invention involves combining information that typically forms electronic medical records (EMR), such as the reason for exam (RFS), e.g., including factors such as demographics, past medical history, medications, laboratory results and the like, as part of the medical imaging order/request, with findings and/or impressions from (retrospective) radiology reports of a large population of patients.

According to the above aspect, features from the EMR and/or radiology text can be used to train a machine-learning model, e.g., a deep learning model, which is configured to process the findings and/or abnormalities stated by a radiologist when the radiologist interprets and diagnoses medical images. The method can then alert and/or recommend that certain additional abnormalities should be considered based on common radiologic presentations of specific diseases, as learned from large volumes of EMR and radiology reports.

The described aspect of the invention thus leverages non-imaging patient data to address the possibility of delays in diagnoses due to satisfaction of search errors when interpreting medical images, thereby providing better context for more accurate reading and interpretation of such images by the radiologist. Experiments have shown that this way, the degree of satisfaction of search errors by the radiologist can be significantly reduced.

As described above, the text data representing the at least one radiologic finding relating to the patient may be obtained by the machine-learning model from the radiologist. For example, the radiologist may dictate his/her finding(s) while diagnosing a medical image of the patient.

As an alternative aspect, the text data representing the at least one radiologic finding relating to the patient which serves as input into the machine-learning model may be automatically generated by an automated analysis of one or more medical images of the patient. For example, an imaging AI model may be used to generate the text data automatically. Accordingly, this aspect of the invention supports an automated reading of medical images by AI models.

In one aspect of the invention, obtaining the text data may comprise receiving the text data through one or more user inputs, e.g. with a keyboard.

Alternatively, obtaining the text data may comprise receiving audio data and automatically performing a speech-to-text conversion to generate the text data. This way, the machine-learning approach of the invention may be directly embedded in the radiologist's workflow when he/she dictates the findings.

As another alternative, obtaining the text data may comprise automatically processing one or more medical images of the patient by an automated image processing system, e.g., an imaging model, to generate the text data. In this variant, the method is directly embedded into an image analysis technique to further automate the medical image processing.

In another aspect of the invention, the machine-learning model may be based on a set of medical concepts extracted from the training data and/or training the machine-learning model may comprise extracting a set of medical concepts from the training data. The medical concepts may refer to information relevant to the medical status of a patient comprised in a radiology report and/or EMR report. The set of medical concepts may comprise medical concepts selected from the group comprising: medical concepts relating to demographics, radiologic findings, past medical history, laboratory results and/or medications.

The machine-learning model may comprise a Transformer model, in particular a Bidirectional Encoder Representations from Transformers, BERT, model. Generally speaking, a Transformer model may be understood as a (deep) learning model that preferably adopts the mechanism of attention, differentially weighing the significance of each part of the input data. Transformer models are used primarily in the field of natural language processing and in computer vision. One characteristic of a Transformer model is that it does not necessarily process the data in order, unlike a recurrent neural network (RNN). Apart from that, the term Transformer is to be understood in line with the common understanding of the person skilled in the art (see e.g., https://en.wikipedia.org/wiki/Transformer_(machine_learning_m odel)).

The training may comprise training the Transformer model, in particular one or more embeddings thereof, using the extracted set of medical concepts. This way, the machine-learning model is particularly suited for processing the radiology and EMR reports, as will be explained further below.

In one aspect of the invention, the Transformer model may be extended by an episode embedding. Preferably, the episode embedding captures which episode of care the data from the radiology reports and/or the data from the electronic medical records belongs to. Preferably, an episode of care relates to a single observation of the patient. This way, the Transformer model is specifically adapted to the application domain at hand, namely the analysis of data relating to medical images.

In another aspect, the Transformer model may comprise a masked language modeling, MLM, function, for which approximately 5% to 25%, more preferably approximately 10% to 20%, and even more preferably approximately 15% of the input are masked as follows:
- an input token is replaced with a mask token (e.g., [MASK]) with a first probability;
- an input token is replaced with one or more random words from a vocabulary of the data from the radiology reports and the data from the electronic medical records with a second probability; and
- an input token is not replaced if, preferably only if, it relates to a radiologic finding with a third probability. Preferably, the first probability is 80%, the second probability is 10% and/or the third probability is 10%. Also in this aspect, the model is specifically adapted to the problem domain to yield optimal results.

In yet another aspect, the Transformer model comprises a current episode prediction, CEP, function configured to identify whether two input sequences belong to the same episode of care. Preferably, an episode of care relates to a single observation of the patient. In this aspect, the model is even more specifically adapted to the problem domain to yield optimal results.

The machine-learning model may comprise a question-answering, QA, model, configured to use one or more embeddings from the Transformer model. This way, it is possible to generate particularly accurate suggested output findings.

The method may further comprise displaying the at least one predicted additional radiologic finding relating to the patient on an electronic display. The method may further comprise: receiving user input indicating that the at least one predicted additional radiologic finding is either accepted or rejected, and if the user input indicates that the at least one predicted additional radiologic finding is accepted, adding the at least one predicted additional radiologic finding to a radiology report of the patient. This way, it is ensured that only findings validated by a human are finally added to the radiology report.

The invention also provides a machine-learning model usable in any of the methods disclosed herein, as well as a method of training the machine-learning model. The training method may use training data, the training data comprising text data from radiology reports and text data from electronic medical records of a plurality of patients.

Also provided is a data processing apparatus or system comprising means for carrying out any of the methods disclosed herein.

Finally, the invention provides a computer program comprising instructions which, when the program is executed by a computer, cause the computer to carry out any of the methods disclosed herein.

The disclosure may be better understood by reference to the following drawings:
Fig. 1: A flow chart showing the data flow for training the model and providing suggestions to the radiologist in accordance with embodiments of the invention.
Fig. 2: An exemplary input representation for pretraining tasks in accordance with embodiments of the invention.

The above mentioned attributes, features, and advantages of this invention and the manner of achieving them, will become more apparent and understandable (clear) with the following description of embodiments of the invention in conjunction with the corresponding drawings. Embodiments of the invention will be described which use EMR and/or radiology report features to minimize "Satisfaction of Search" errors in medical image interpretation.

In the following, an embodiment of the invention for training a machine-learning model and using the model to suggest additional radiologic findings to the radiologist will be presented. It will be appreciated that the aspects relating to the training of the model may be executed independently, possibly by a different entity, than the aspects relating to the use of the trained model. It will also be appreciated that radiology is just one of various exemplary application domains for the principles disclosed herein. The person skilled in the art will readily understand that the principles and aspects disclosed herein may be applied to any type of medical domain and may involve any type of medical record, medical image and medical practitioner, as long as the respective domain lends itself to the principles disclosed.

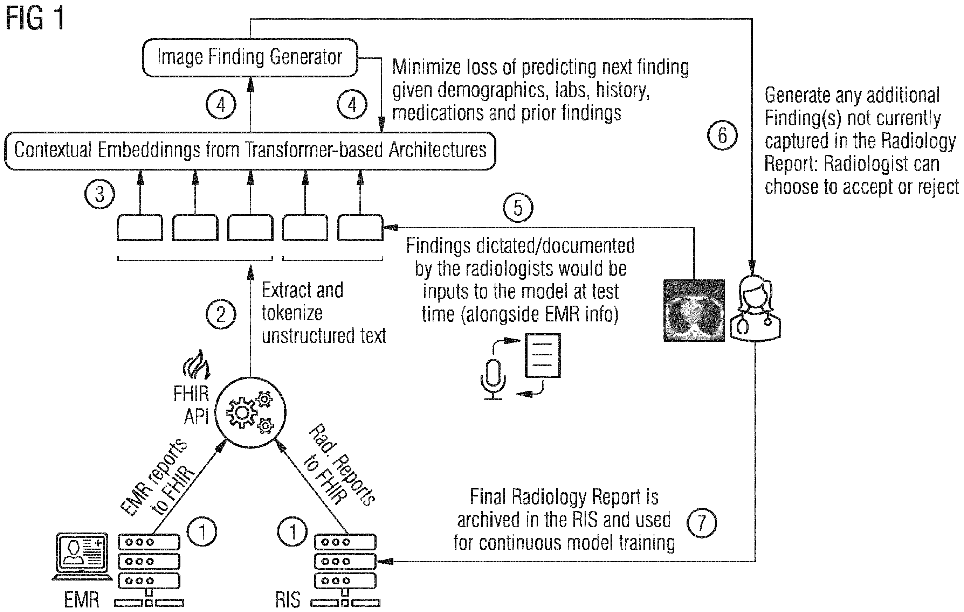

In the embodiment illustrated in Fig. 1, the overall input for training the machine-learning model includes one or more electronic medical record (EMR) reports and one or more radiology reports. To obtain a sufficient degree of accuracy, the machine-learning model may be trained with a fast number of EMR reports and radiology reports, such as 10,000 or more sets of EMR report/radiology report combinations. In a preferred embodiment, only textual information is used from both sources. For example, relevant data from the EMR reports which represents the respective reason for exam (RFS), including factors such as e.g., demographics, past medical history, medications, laboratory results and the like, may be used on the one hand, and data describing one or more radiologic findings from the radiology reports on the other hand.

This information serves as training data, i.e. as input data suitable for training the machine-learning model of embodiments of the invention. In the following, exemplary concrete data sets suitable as training data are presented, including an exemplary EMR report and an exemplary radiology report:
Exemplary radiology report:
   "Extensive bilateral left greater than right infiltrates with cavitary process, left perihilar area and probable abscess left lower lobe. Left hilar adenopathy suspected."
Exemplary EMR report:
   "55y/o F with a past medical history of metastatic breast ca to the brain s/p craniotomy and s/p R total hip arthroplasty who is presenting with two days of increasing lethargy. Review of systems is also positive for cough, abdominal cramping and diarrhea. She denies any headaches, changes in vision, chest pain, vomiting,
   or rashes. In the emergency department, she was febrile to 101.8F, with a leukocytosis up to 15 and Lactate was 1."

In step 1 of Fig. 1, the (text) data from the EMR reports and radiology reports are mapped to FHIR (Fast Healthcare Interoperability Resources). Preferably, the entire data from the EMR reports and radiology reports is mapped to FHIR.

As the person skilled in the art knows, FHIR is a standard created by the Health Level Seven International (HL7) healthcare standards organization which defines data formats and elements (called "resources") and an application programming interface (API) for exchanging electronic health records (EHR). In one embodiment, the radiology report may be mapped to the DiagnosticReport resource. The EMR report may be mapped to a combination of resources, e.g., Observation, Condition, ClinicalImpression, MedicationStatement and/or Procedure.

It shall be understood that FHIR is only one example of a concrete standardized format. What is relevant in step 1 is that the input data is mapped into a standardized format that is suitable for being processed in the subsequent steps. FHIR is nowadays in wide-spread use and one of the de-facto standards for interoperability between EMR systems across hospitals, while enabling the export of such data for secondary research. However, the principles of the invention can still be implemented without FHIR, with the requirement of access to the free text EMR and/or radiology reports in the EMR systems.

In one embodiment, the mapping to FHIR can be performed by one or more Open-Source scripts and/or proprietary scripts. Examples include Pyrog (https://github.com/arkhn/pyrog) and firely FHIRMapper (https://docs.fire.ly/projects/FHIRMapper/start.html).

In step 2 of Fig. 1, the FHIR data is then tokenized and fed to an information extraction model to detect and extract relevant (e.g. pre-defined) medical concepts. In one embodiment, the extracted medical concepts are selected from the group comprising demographics, radiologic findings, past medical history, lab results and/or medications.

In step 3, the extracted medical concepts are then encoded and used to train and/or fine-tune a pre-trained machine-learning model. In one embodiment, the machine-learning model is based on a Transformer architecture and the (pre-)training concerns one or more contextual embeddings of the model.

In one particular embodiment, a bidirectional encoder representations from transformers (BERT) architecture is used. As the person skilled in the art will appreciate, BERT is a transformer-based machine-learning technique for natural language processing (NLP) pre-training. Open-source implementations are available e.g., at https://github.com/google-research/bert or https://github.com/hanxiao/bert-as-service. It shall be noted that other architectures, e.g. LSTM (long short term memory) and/or RNNs (recurrent neural networks), or more generally any suitable transformer architecture could be used in place of BERT-based approaches in other embodiments of the invention.

In some embodiments, BERT as a service may be used, which can be run in parallel on separate GPUs to speed up the fine-tuning (see e.g. https://bert-as-service.readthedocs.io/en/latest/section/faq.html#what-is-the-parallel-processing-model-behind-the-scene).

In one embodiment of the invention, in addition to the token, segment and position embeddings in a standard-implementation of BERT, which are normally summed up as the input representation for BERT, the BERT architecture is extended with an additional embedding, namely episode embedding. The episode embedding may capture which episode of care the EMR and/or radiology information mentioned further above belongs to. An episode of care may refer to all or at least a subset of details on the physical examination, diagnostic procedures and/or treatment performed in a single medical investigation, such as a single in-patient or out-patient visit. This way, the BERT architecture is extended and specifically adapted to the target domain (interpretation of medical findings).

In one embodiment of the invention, either in addition or alternatively to the above-explained extension of BERT, the masked language modeling (MLM) and/or the next sentence prediction (NSP) pretraining tasks for BERT may be modified to accomplish the task of predicting the next medical (e.g., radiology) finding:
With respect to the input sequence of tokens for the MLM task, 15% of radiology and EMR derived tokens are masked at random in one embodiment: 80% of the time, they would be replaced by the [MASK] tag, 10% of the time they would be replaced by random words from the vocabulary of EMR and radiology tokens, and 10% of the time, radiology findings (only) would not be replaced. This is to maximize bias towards predicting observed radiology findings (not EMR information).

The inventors have found that the above ratios result in a particularly suitable model. However, it shall be appreciated that variations from these values are possible within the scope of the invention.

Concerning BERT's NSP function, instead of predicting if two random sequences from the training data are related, the NSP is revised to CEP (current episode prediction) in one embodiment, i.e. a binarized task to identify if two sequences belong to the same episode of care or not. In one embodiment, 50% of the time the sequences would come from reports belonging to the same episode.

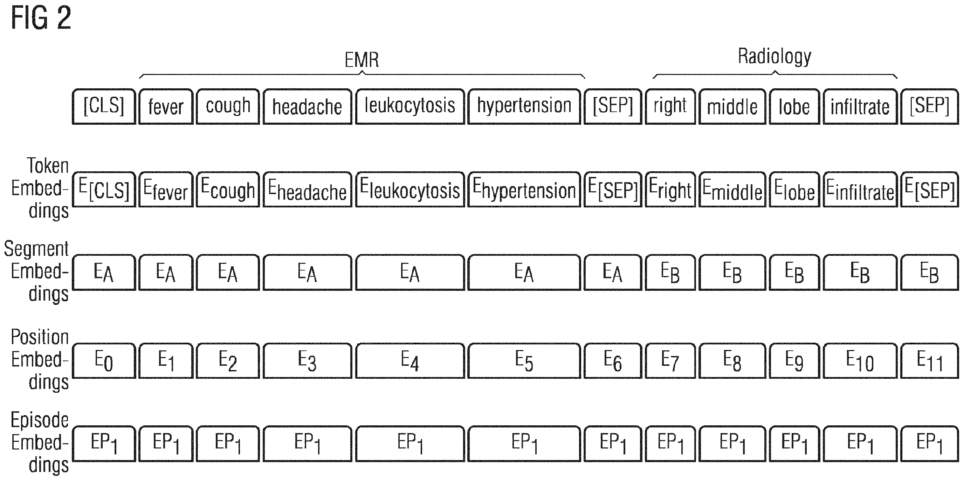

For example, Fig. 2 shows an exemplary sequence of medical concepts extracted from an EMR report:
- "fever" (representing a symptom)
- "cough" (representing a symptom)
- "headache" (representing a symptom)
- "leukocytosis" (representing laboratory data)
- "hypertension" (representing past medical history)

In addition, Fig. 2 shows an exemplary sequence of medical concepts extracted from a radiology report: "right", "middle", "lobe", "infiltrate" representing findings.

Since both reports were documented during one in-patient visit in this example, they have the same or a similar episode embedding (EP1), as shown in Fig. 2, and would thus be learned as part of the CEP task.

In the embodiment illustrated in Fig. 1, in step 4 the embeddings from the model pretrained to accomplish the modified MLM and CEP tasks are fed as input into a question answering (QA) model (denoted as "Image Finding Generator" in Fig. 1). In one embodiment, step 4 is a machine-learning process in which the cost function of the model architecture (the Image Finding Generator) is minimized according to the correctness of a current radiologic finding prediction, which is driven by the models' representation of the prior predicted finding combined with the EMR-derived context (demographics, past medical history, etc.), i.e. the contextual embeddings.

In one embodiment, the QA model is trained on a multi-slot filling task. In other words, at test time, i.e. when a radiologist deals with a particular patient, a sequence (i.e., 0 ... n) of radiology findings documented by the radiologists as well as relevant information extracted from an associated EMR report of the patient can be provided as input to the model with the assumption that some radiology findings (i.e. slots) are missing. This is illustrated in step 5 of Fig. 1.

As an example, the input could be (fever, cough, headache, leukocytosis, appendectomy, right middle lobe infiltrate, X1, X2). X1 and X2 are then predicted by the model (e.g. X1 = rib fracture?, X2 = hilar adenopathy?, with "?" denoting that the model is suggesting if the radiologist should check for these findings, not diagnosing that the findings exists in the image.

More generally speaking, the model generated one or more additional suggested findings (see step 6 in Fig. 1) which are not included in the radiologist's radiology report. The radiologist may then choose to accept or reject the suggested finding(s).

If accepted, the one or more findings may be added to the radiology report (see step 7 in Fig. 1). Furthermore, the one or more findings added to the radiology report may be used for training the model in the future.

In one embodiment of the invention, the overall method described above may be directly embedded in the image analysis process performed by the radiologist, either on the input side, the output side, or both, as follows:
On the input side, the radiologist may inspect a given set of medical images, e.g. from an X-ray radiography, computed tomography (CT), magnetic resonance imaging (MRI) or the like, of a patient. The radiologist may dictate one or more findings as part of his/her diagnosis of the images, e.g., using an electronic device. The dictated audio data may then be, preferably automatically, converted to text, e.g. using speech-to-text functionality known to the person skilled in the art. The resulting text may then serve directly as text data representing at least one radiologic finding of the patient, which may be directly input into the machine-learning model, as explained further above in connection with step 5 of Fig. 1.

Another real-world application for embodiments of the invention may involve improving the results of an automated AI model that analyzes radiologic (e.g. X-ray, CT and/or MRI) images while a radiologist is reviewing the same images, towards helping the radiologist generate more accurate impressions assisted by the imaging AI model that is augmented by EMR context and suggested radiologic findings.

On the output side, the generated at least one predicted additional radiologic finding relating to the patient may be output directly on a screen on which the radiologist also observes the original set of medical images. In other words, the method of this embodiment of the invention creates a closed loop and gives the radiologist an almost immediate feedback to his/her input (e.g., dictation) of his/her one or more findings.

While being recommendations in the above embodiment, the computer-generated suggested findings could also be used during quality improvement initiatives and/or training seminars to bring certain errors (such as satisfaction of search) to the attention of radiologists who may be less experienced and did not review the images that were processed by the system.

In the following, a preferred embodiment of the above-described methods will be illustrated by way of concrete examples:
The following is a sample from a typical radiology report (specifically the reason for exam):
   "Please evaluate inferior pelvis (pectineus muscle) for fluid collection noted on previous non-contrast ct. R/o infection vs hematoma. No contraindications for iv contrast"
The following is a sample from an EMR report (specifically with history of present illness):
   1. 55 F with a pmh s/f metastatic breast ca to the brain s/p craniotomy and bone s/p R THA who is presenting with two days of increasing lethargy
   2. In the emergency department, she was febrile to 101.8F
   3. A CXR was negative for PNA, a UA was notably positive, and the patient was noted to have erythema and edema around her right hip, clinically consistent with cellulitis vs osteomyelitis vs septic arthritis
The following is a sample from an EMR report (specifically with past medical history):
   1. Breast carcinoma diagnosed as invasive ductal breast ca, BRCA1 positive, s/p surgical management with chemotherapy and tamoxifen. Recurrence,
      bony mets to right hip s/p THA and brain s/p whole brain radiation. Also had Taxol and Herceptin. Recurrance with brain mets s/p left craniotomy, and right shoulder mets, currently on Tykerb
   2. Rheumatoid Arthritis
   3. Patent foramen ovale -s/p cva with right sided visual loss, anticoagulated on coumadin

As mentioned earlier, the input data is preferably mapped to the FHIR format. A generic example of a FHIR document can be found at https://www.hl7.org/fhir/document-example-dischargesummary.xml.html.

From the input data or the FHIR document, respectively, the following medical concepts are extracted in the example:
From the reason for exam:
   1. Infection suspected
   2. Hematoma suspected
   3. Fluid collection suspected
From the history of present illness:
   1. Metastatic cancer
   2. Breast cancer, primary
   3. Sleepiness, excessive
   4. Craniotomy
   5. Fever
   6. Septic arthritis
   7. X-ray done
   8. Erythema
   9. Cellulitis
   10. Osteomyelitis
From the past medical history:
   1. Breast cancer, primary
   2. Surgery
   3. Chemotherapy
   4. Arthroplasty
   5. Cancer metastatic to bone
   6. Radiotherapy
   7. Cancer metastatic to brain
   8. Craniotomy
   9. Rheumatoid arthritis
   10. Patent foramen ovale
   11. Visual impairment, right
   12. Stroke
   13. Anticoagulation

The following table shows exemplary inputs and outputs of the image finding generator:

| Input findings | Suggested (output) findings |
|---|---|
| <none> | 1. Lesion, enhancing |
| | 2. Metastatic cancer |
| 1. Lesion, enhancing | Check pathological fracture |
| 2. Metastatic cancer | |

As can be seen, the radiologist has dictated "Lesion, enhancing" and "Metastatic cancer" (input findings) as his findings while investigating the patient's medical image. These findings are then also included in the suggested output findings, along with an additional suggested finding generated by the computer, namely "Check pathological fracture". The optional expression "Check" in the suggested output findings serves to indicate that this finding is computer-generated and should be confirmed or rejected by the radiologist.

In summary, embodiments of the invention have at least the following technical advantages:
They may leverage non-imaging patient data to address the possibility of delays in diagnoses due to satisfaction of search errors when interpreting medical images, thereby providing better context for more accurate automated reading of such images by AI models.
They may enable the representation of non-imaging information (e.g. EMR and radiology text) by modifications of the BERT pretraining tasks to maximize bias towards predicting observed radiology findings, and/or to predict sequences of tokens related to the same episode of care (current episode prediction/CEP). These tasks may provide the contextual embeddings that can be learned to better suggest relevant radiology findings and support automated reading.
They may improve the performance of digital health products by suggesting possible radiology findings to prevent satisfaction of search errors and minimize delays in the radiologist's diagnoses.

While the invention has been illustrated and described in detail with the help of a preferred embodiment, the invention is not limited to the disclosed examples. Other variations can be deducted by those skilled in the art without leaving the scope of protection of the claimed invention.

## Claims

1. A computer-implemented method for predicting radiologic findings, comprising:
obtaining (5), by a machine-learning model, text data representing at least one radiologic finding relating to a patient and, optionally, text data from an electronic medical record of the patient; and
generating (6), by the machine-learning model, at least one predicted additional radiologic finding relating to the patient;
wherein the machine-learning model is based on training data, the training data comprising text data from radiology reports and text data from electronic medical records of a plurality of patients.

2. The method of claim 1, wherein obtaining the text data comprises:
- receiving the text data through one or more user inputs; or
- receiving audio data and automatically performing a speech-to-text conversion to generate the text data; or
- automatically processing one or more medical images of the patient by an automated image processing system to generate the text data.

3. The method of any of the preceding claims, wherein the machine-learning model is based on a set of medical concepts extracted from the training data and/or wherein training the machine-learning model comprises extracting (2) a set of medical concepts from the training data;
wherein, preferably, the set of medical concepts comprises medical concepts selected from the group comprising: medical concepts relating to demographics, radiologic findings, past medical history, laboratory results and/or medications.

4. The method of any of the preceding claims, wherein the machine-learning model comprises a Transformer model, in particular a Bidirectional Encoder Representations from Transformers, BERT, model.

5. The method of claims 3 and 4, wherein the training comprises training the Transformer model, in particular one or more embeddings thereof, using the extracted set of medical concepts.

6. The method of any one of claims 4 or 5, wherein the Transformer model is extended by an episode embedding;
wherein, preferably, the episode embedding captures which episode of care the data from the radiology reports and/or the data from the electronic medical records belongs to;
wherein, preferably, an episode of care relates to a single observation of the patient.

7. The method of any one of claims 4 to 6, wherein the Transformer model comprises a masked language modeling, MLM, function, for which approximately 5% to 25%, more preferably approximately 10% to 20%, and even more preferably approximately 15% of the input are masked as follows:
- an input token is replaced with a mask token with a first probability;
- an input token is replaced with one or more random words from a vocabulary of the data from the radiology reports and the data from the electronic medical records with a second probability; and
- an input token is not replaced if, preferably only if, it relates to a radiologic finding with a third probability;
wherein, preferably, the first probability is 80%, the second probability is 10% and/or the third probability is 10%.

8. The method of any one of claims 4 to 7, wherein the Transformer model comprises a current episode prediction, CEP, function configured to identify whether two input sequences belong to the same episode of care;
wherein, preferably, an episode of care relates to a single observation of the patient.

9. The method of any one of claims 4 to 8, wherein the machine-learning model comprises a question-answering, QA, model, configured to use (4) one or more embeddings from the Transformer model.

10. The method of any of the preceding claims, further comprising:
displaying the at least one predicted additional radiologic finding relating to the patient on an electronic display.

11. The method of claim 10, further comprising:
receiving user input indicating that the at least one predicted additional radiologic finding is either accepted or rejected; and
if the user input indicates that the at least one predicted additional radiologic finding is accepted, adding the at least one predicted additional radiologic finding to a radiology report of the patient.

12. A machine-learning model usable in the method of any one of claims 1 to 11.

13. A method of training the machine-learning model of claim 12 using training data, the training data comprising text data from radiology reports and text data from electronic medical records of a plurality of patients.

14. A data processing apparatus or system comprising means for carrying out the method of any one of claims 1 to 11.

15. A computer program comprising instructions which, when the program is executed by a computer, cause the computer to carry out the method of any one of claims 1 to 11.
